# EUROPEAN PATENT APPLICATION

(11) **EP 2 742 895 A2**
(43) Date of publication of application: **18.06.2014**
(21) Application number: 13197498.2
(22) Date of filing: 16.12.2013
(51) Int. Cl.: A61B 19/00, G06F 19/00

(54) **Recognizing which instrument is currently active**

(30) Priority: 17.12.2012 US 201213716550
(71) Applicant: Biosense Webster (Israel), Ltd., Yokneam 20692 (IL)
(72) Inventor: Ludwin, Doron Moshe, 32207 Haifa (IL); Zino, Eliahu, 30300 Atlit (IL)
(74) Representative: Carpmaels & Ransford LLP

(57) **Abstract**

A medical device includes an insertion tube, having a proximal end and a distal end, which is configured for insertion into a body of a patient. A handle is fixed to the proximal end of the insertion tube and is configured to be held by a user of the device. A sensor associated with the handle is configured to generate a signal to a control console indicating whether the user is holding the handle.

## Description

### FIELD OF THE INVENTION

The present invention relates generally to invasive medical devices, and particularly to methods and apparatus for user interaction with such devices.

### BACKGROUND

In some minimally-invasive medical procedures, multiple instruments may be inserted percutaneously into a given organ or body cavity and may be in operation there simultaneously. For example, the CARTO® 3 System, produced by Biosense Webster Inc. (Diamond Bar, California), is capable of concurrently tracking and visualizing multiple catheters inside the heart. The catheters may be used for various diagnostic and therapeutic purposes, such as ablating myocardial tissue to treat atrial fibrillation or other conditions, as well as measuring intracardiac electrogram signals at different locations before, during and after the ablation. The CARTO system is capable of creating and displaying a three-dimensional (3D) map of the heart and showing on the map the positions of the catheters and even of the individual electrodes on the catheters that are used to collect the electrogram signals. When the operator clicks a particular electrogram trace on the display screen, the corresponding electrode may be identified.

### SUMMARY

Embodiments of the present invention that are described hereinbelow provide apparatus and methods to facilitate operator control of invasive medical devices.

There is therefore provide, in accordance with an embodiment of the present invention, a medical device, including an insertion tube, having a proximal end and a distal end, which is configured for insertion into a body of a patient. A handle is fixed to the proximal end of the insertion tube and is configured to be held by a user of the device. A sensor associated with the handle is configured to generate a signal to a control console indicating whether the user is holding the handle.

In a disclosed embodiment, the sensor includes a flexible strain sensor that is fitted over an outer surface of the handle.

Alternatively, the sensor may be configured to detect that the user is holding the handle responsively to movement of the handle. In one such embodiment, the insertion tube contains a first magnetic field sensor in the distal end, for use in detecting a position of the distal end within the body responsively to a magnetic field applied from outside the body, and the sensor in the handle includes a second magnetic field sensor, which generates the signal in response to the magnetic field.

In some embodiments, the insertion tube is configured for insertion into a heart of the patient, and the handle includes one or more controls for operation by the user in manipulating the distal end of the insertion tube within the heart.

There is also provided, in accordance with an embodiment of the present invention, medical apparatus, including a plurality of invasive probes. Each such probe includes an insertion tube, having a proximal end and a distal end, which is configured for insertion into a body of a patient, and a handle, which is fixed to the proximal end of the insertion tube and is configured to be held by a user of the probe. A sensor in each probe is configured to generate a signal indicating whether the user is holding the handle. The apparatus also includes a display and a console, which is configured to present information on the display with respect to operation of the invasive probes inside the body and to provide, responsively to the signal from each probe, an indication as to which of the probes is currently being held by the user.

In some embodiments, the console is configured to present on the display an image of an organ in the body including symbols indicating respective positions of the probes in a vicinity of the organ, and the indication includes a change with respect to the symbols on the display to designate at least one of the probes that is currently being held by the user. The image may include a three-dimensional view of the organ, wherein the indication includes rotating the three-dimensional view so as to enhance a visibility of a symbol corresponding to the at least one of the probes on the display. Alternatively or additionally, the console may be configured to detect position coordinates of the probes in the body and to generate the map based on the detected position coordinates of the at least one of the probes that is currently being held by the user.

In one embodiment, the indication includes an audible notification identifying at least one of the probes that is being held by the user.

In another embodiment, at least one of the probes includes a functional element configured to apply a treatment to tissue in the body, and the console is configured to interrupt the treatment upon detecting that the user is not current holding the at least one of the probes. Two or more of the probes may be configured to apply the treatment, and the console may be configured to select one of the probes for application of the treatment by detecting that the one of the probes is currently being held by the user.

In yet another embodiment, the probes include a functional element configured to receive physiological signals from tissue in the body, and the console is configured to present the physiological signals on the display, wherein the indication includes highlighting on the display at least one of the physiological signals that originates from one of the probes that is currently being held by the user.

There is additionally provided, in accordance with an embodiment of the present invention, a method for controlling a medical procedure, which includes providing a plurality of invasive probes for concurrent insertion into a body of a patient, each such probe including a handle, which is configured to be held by a user of the probe. Information is presented on a display with respect to operation of the invasive probes inside the body, the information including an indication as to which of the probes is currently being held by the user.

In disclosed embodiments, presenting the information comprises receiving a signal from a respective sensor associated with the handle of each of the probes, indicating whether the user is currently holding the handle, and generating the indication responsively to the signal. Receiving the signal may include sensing pressure exerted by a hand of the user on the handle and/or sensing a movement of the handle.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for cardiac catheterization, in accordance with an embodiment of the present invention;
Fig. 2 is a schematic top view of a set of catheters in use during a medical procedure, in accordance with an embodiment of the present invention;
Fig. 3 is a schematic representation of a display screen presented by a cardiac catheterization system, in accordance with an embodiment of the present invention; and
Fig. 4 is a schematic side view of a pressure-sensitive sleeve being fitted onto a catheter handle, in accordance with an embodiment of the present invention.

### DETAILED DESCRIPTION OF EMBODIMENTS

When multiple invasive devices are inserted concurrently into a patient's body during a minimally-invasive medical procedure, with only the proximal ends and handles of the devices protruding outside the body, it may be difficult for the user (such as an operating physician) to know which handle belongs to which device. This problem can be particularly troublesome in cardiac catheterization procedures, in which multiple catheters may be in concurrent use inside the heart. The embodiments that are described below therefore relate specifically to such cardiac procedures. The principles of the present invention, however, may also be applicable to other sorts of treatments, both therapeutic and diagnostic, using various sorts of invasive medical devices in various regions and organs inside the body.

The disclosed embodiments make use of an invasive probe, such as a catheter, comprising an insertion tube, whose distal end is inserted into the body of a patient. A handle is fixed to the proximal end of the insertion tube. A sensor associated with the handle generates a signal to a control console, indicating whether the user is holding the handle. The sensor may comprise, for example, a flexible strain sensor that is fitted over the outer surface of the handle and responds to pressure of the user's hand, or alternatively a sensor that responds to movement of the handle.

The console presents information on a display with respect to operation of multiple invasive probes of this sort inside the body, including an indication as to which probe the user is currently holding at any given time during the procedure based on the signals received from the handle sensors. The console may display an image of an organ in the body with symbols, such as icons, indicating respective positions of the probes in the vicinity of the organ, and may change the way the symbols are displayed in order to designate the probe (or probes) that is currently being held by the user. As one example, the console may rotate a 3D view of the image of the organ (such as a 3D map of the organ) so as to enhance the visibility of the symbol corresponding to the probe that the user is holding. Alternatively or additionally, the console may give an audible identification of the probe that is being held by the user.

Further additionally or alternatively, upon determining which of the probes the user is currently holding, the console may use this information in controlling and/or displaying the results of a diagnostic or therapeutic treatment. For example, the console may select one of the probes to actuate for application of a treatment by detecting that the user is holding the probe. Concomitantly, the console may interrupt treatment applied by one of the probes upon detecting that the user is not currently holding it. As another alternative, when the probes receive physiological signals from tissue in the body, and the console presents the physiological signals on a display, the console may highlight the displayed signals that originate from the probe that is currently being held by the user.

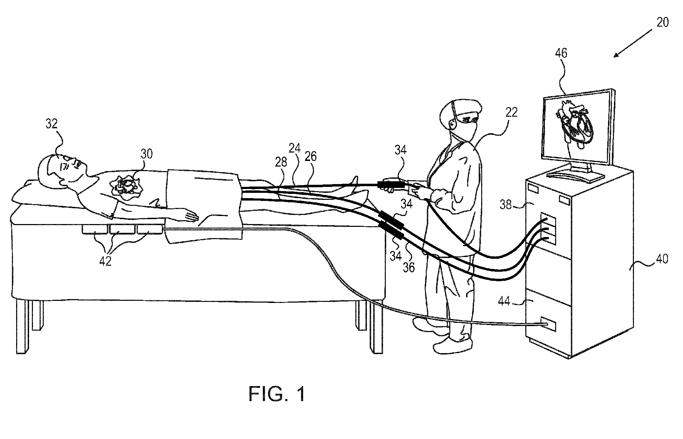

Fig. 1 is a schematic pictorial illustration of a system 20 for invasive treatment of a heart 30 of a patient 32, in accordance with an embodiment of the present invention. An operating physician (referred to simply as an operator 22, or equivalently as a user of the system), such as a cardiologist, inserts flexible probes, such as catheters 24, 26 and 28, through the vascular system of patient 32 so that the distal ends of the catheters enter a chamber of the patient's heart. Operator 22 may advance the catheters so that their distal tips engage endocardial tissue at desired locations. The operator manipulates each catheter using a handle 34 at its proximal end. Typically, the user holds only one of the handles at any given time, and simply rests the unused handles in a convenient location.

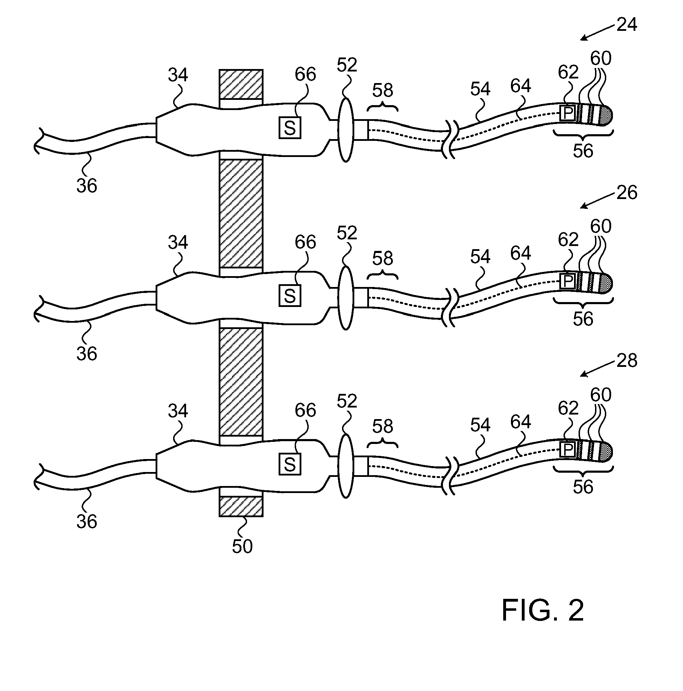

Catheters 24, 26, 28 are connected via cables 36 at their proximal ends to a console 40. The console comprises a control unit 38, which sends and receives signals to and from the catheters in order to control and monitor their operations. For example, the control unit may apply electrical energy to catheter 24 in order to ablate tissue in heart 30 and/or may receive electrical signals from the catheters in order to measure cardiac electrical activity. Alternatively or additionally, catheters 24, 26 and 28 may be used for other diagnostic and/or therapeutic functions, as are known in the art. In addition, control unit 38 receives position signals from sensors in the distal ends of the catheters (shown in Fig. 2), which enable the control unit to determine position coordinates of the distal ends within the body. These position coordinates may be used, *inter alia,* in tracking the locations of the catheters and in mapping the interior of the heart, as in the above-mentioned CARTO system, for example.

In the embodiment shown in Fig. 1, system 20 uses magnetic position sensing to determine position coordinates of catheters 24, 26 and 28. For this purpose, a driver circuit 44 in console 40 drives field generators 42 to generate magnetic fields within the body of patient 32. Typically, field generators 42 comprise coils, which are placed below the patient's torso at known positions external to the body. These coils generate magnetic fields in a predefined working volume that contains heart 30. Magnetic transducers (such as miniature coils) within the distal ends of the catheters output electrical signals in response to these magnetic fields. Control unit 38 processes these signals in order to determine position (location and/or orientation) coordinates of the distal ends of the catheters. Console 40 may use the coordinates of the catheters in driving a display 46, particularly to show the locations and status of the catheters, which are typically superimposed on an map or other image of the heart.

Methods of magnetic position sensing and processing that may be applied by control unit 40 in finding the coordinates of catheters 24, 26 and 28, using the magnetic transducers in the catheters, are described in detail, for example, in U.S. Patent 6,690,963, whose disclosure is incorporated herein by reference. Alternatively or additionally, the methods described hereinbelow may be applied using position transducers of other types, such as impedance-based or ultrasonic position sensors. (The term "position transducer" as used herein refers to any element that causes console 40 to receive signals indicative of the coordinates of the element.) Furthermore, as noted earlier, the methods described hereinbelow may similarly be applied in diagnostic and therapeutic applications using not only catheters, but also invasive probes of other types, both in the heart and in other body organs and regions.

Fig. 2 is a schematic top view of catheters 24, 26 and 28, showing functional details of the catheters of relevance to embodiments of the present invention. In this embodiment, it is assumed that all three catheters are in concurrent use in an invasive cardiological procedure, but rest on a stand 50 when not actually held by operator 22. Each catheter comprises a flexible insertion tube 54, having a distal end 56 that is inserted into heart 30, and a proximal end 58 that is attached to handle 34. A control 52 on the handle (or multiple controls) enables the user to steer and possibly to control other functions of distal end 56.

Catheters 24, 26, 28 comprise one or more functional elements - exemplified in this embodiment by electrodes 60 - and a position transducer 62 in their distal ends 56. Electrodes 60 may be used either for diagnostic or therapeutic purposes, or both, such as sensing electrogram signals in the body, pacing the heart, or ablating heart tissue by application of radio-frequency (RF) electrical energy. Position transducer 62, in the form of a coil, for example, outputs position signals in response to the magnetic fields of field generators 42. Alternatively, as noted earlier, the position transducer may be based on any suitable sort of position sensing technology that is known in the art. Electrodes 60 and position transducer 62 communicate via wires 64 through insertion tube 54 and cable 36 with control unit 38.

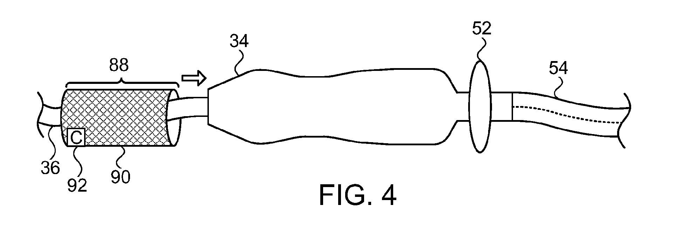

Each handle 34 comprises a sensor 66, which generates a signal to console 40 indicating whether or not operator 22 is holding the handle at any given time. Sensor 66 may comprise, for example, a flexible strain sensor, which senses pressure exerted by the operator in grasping the handle, as is described below with reference to Fig. 4. Alternatively, sensor 66 may sense movement of handle 34, which may comprise continual movement as the operator moves his or her hand while holding the handle, as well as a single movement, when the operator lifts the handle from stand 50. For this purpose, sensor 66 may comprise, for example, a magnetic transducer, similar in principle to transducers 62, which generates the signal to the console in response to the magnetic fields of field generators 42. Console 40 processes this signal in order to determine the location of handle 34, and thus whether the handle is located on stand 50 or is being held away from the stand by the operator.

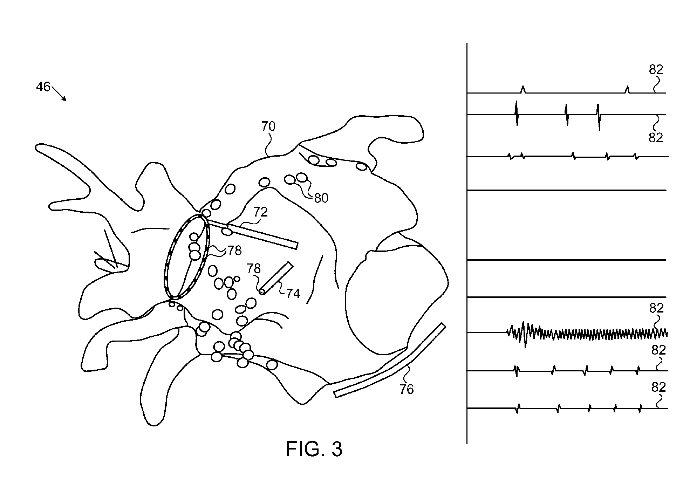

Console 40 may use the information provided by sensor 66 in automatically indicating to operator 22 which of catheters 22, 24 and 26 is currently active, i.e., which of the catheters the operator is currently holding. This indication may be visual, as illustrated in Fig. 3, for example, and described with reference to this figure hereinbelow. Alternatively or additionally, the console may give an audible indication. For example, each catheter may have a pre-assigned name, and the console may announce the name of the catheter that the operator is holding using a suitable speech synthesis function and audio speaker (not shown).

Additionally or alternatively, console 40 may use the indication provided by sensor 66 in controlling aspects of a procedure carried out by system 20. For example, if console 40 detects that operator 22 is not currently holding the catheter that is supposed to be applying some treatment (therapeutic or diagnostic) to tissue in heart 30 during a procedure, the console may automatically interrupt the treatment in order to avoid undesired and possibly dangerous results. Additionally or alternatively, console 40 may automatically select one of the catheters for application of the treatment by detecting that the operator is holding the particular catheter.

Fig. 3 is a schematic representation of display 46 in system 20, in accordance with an embodiment of the present invention. The display shows a 3D map 70 of a chamber of heart 30, on which symbols, in the form of icons 72, 74 and 76, are superimposed to indicate the positions of the distal ends of the corresponding catheters in the heart. (Icon 72 in this example represents the arcuate end section of a "lasso" catheter, as is known in the art.) Marks 78 on icons 72, 74 may correspond to the actual locations of electrodes 60 in heart 30, while marks 80 on the surface of the heart chamber indicate locations that the electrodes have contacted and, possibly, treated (by RF ablation, for example).

Console 40 generates map 70, as in the CARTO system, by detecting and recording the coordinates of the distal tip of one or more of catheters 24, 26 and 28 as the tip contacts the endocardium at different locations in the heart. Typically, the console continues to build and refine the map over the course of at least part of the procedure during which the catheters are inserted into the heart. Based on the signals from sensors 66, the console may automatically choose the catheter that is to serve as the mapping catheter at any point during the procedure, i.e., it may generate the map based on the detected position coordinates of the catheter that is currently being held by the user.

Typically, console 40 may change the appearance of icons 72, 74 and 76 on display 46 to indicate the catheter that the operator is currently holding. For example, the console may visually highlight the icon corresponding to this catheter. Additionally or alternatively, the console may rotate the 3D view of map 70 that is shown in the display so as to enhance the visibility of the icon corresponding to the catheter that the operator is holding. For example, the view angle of the map may be rotated so that the icon in question is located at the front and center of the display.

Display 46 in Fig. 3 also shows traces of physiological signals 82, in this case electrogram signals that are received by electrodes 60. Traces 82 may be visually keyed to marks 78 (by color, for example) to indicate the association between signal traces and electrodes. Console 40 may modify the appearance of signals 82 to highlight the signals originating from the electrodes on the catheter that the operator is currently holding. For example, these particular signal traces may be moved to the top of the display pane or marked on the display by changing their color, brightness or other features.

Fig. 4 is a schematic side view of a pressure-sensitive sleeve 88 being fitted onto catheter handle 34, in accordance with an embodiment of the present invention. Sleeve 88 in this embodiment performs the function of sensor 66 in the embodiment shown in Fig. 2. Sleeves of this sort may be fitted over some or all of the catheters that are connected to console 40, and a registration procedure may be carried out, if necessary, before beginning treatment of the patient to indicate to the console the association between sleeves and catheters.

Sleeve 88 comprises a flexible strain sensor 90, which is fitted over the catheter handle so as to detect pressure exerted by the operator's hand in grasping the handle. Sensor technology that may be used for this purpose is described, for example, by Cochrane et al., in "Design and Development of a Flexible Strain Sensor for Textile Structures Based on a Conductive Polymer Composite," Sensors (2007, 7), pages 473-492, which is incorporated herein by reference. Sleeve 88 may be fitted over the catheter handle either as a permanent fixture or as a removable component, which may be single-use or reusable. A miniature control and communications unit 92 on sleeve 88 receives signals from strain sensor 90 and indicates to console 40, via cable 36 or via a wireless link, whether the operator is holding the catheter handle or has released it, depending on the pressure exerted (or not exerted) by the operator's hand in grasping the handle.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

### Aspects of the invention not yet claimed:

Aspect 1. A method for controlling a medical procedure, comprising:
   providing a plurality of invasive probes for concurrent insertion into a body of a patient, each such probe comprising a handle, which is configured to be held by a user of the probe; and
   presenting information on a display with respect to operation of the invasive probes inside the body, the information comprising an indication as to which of the probes is currently being held by the user.
Aspect 2. The method according to aspect 1, wherein presenting the information comprises receiving a signal from a respective sensor associated with the handle of each of the probes, indicating whether the user is currently holding the handle, and generating the indication responsively to the signal.
Aspect 3. The method according to aspect 2, wherein receiving the signal comprises sensing pressure exerted by a hand of the user on the handle.
Aspect 4. The method according to aspect 2, wherein receiving the signal comprises sensing a movement of the handle.
Aspect 5. The method according to aspect 4, wherein the signal is generated by the sensor responsively to a magnetic field applied from outside the body.
Aspect 6. The method according to aspect 1, wherein presenting the information comprises displaying an image of an organ in the body including symbols indicating respective positions of the probes in a vicinity of the organ, and wherein the indication comprises a change with respect to the symbols on the display to designate at least one of the probes that is currently being held by the user.
Aspect 7. The method according to aspect 6, wherein the image comprises a three-dimensional view of the organ, and wherein the indication comprises rotating the three-dimensional view so as to enhance a visibility of a symbol corresponding to the at least one of the probes on the display.
Aspect 8. The method according to aspect 6, wherein the image comprises a three-dimensional map of the organ, and wherein presenting the information comprises detecting position coordinates of the probes in the body, and generating the map based on the detected position coordinates of the at least one of the probes that is currently being held by the user.
Aspect 9. The method according to aspect 1, and comprising issuing an audible notification identifying at least one of the probes that is being held by the user.
Aspect 10. The method according to aspect 1, wherein presenting the information comprises receiving physiological signals from tissue in the body using the probes, and presenting the physiological signals on the display while highlighting on the display at least one of the physiological signals that originates from one of the probes that is currently being held by the user.
Aspect 11. The method according to aspect 1, wherein providing the plurality of invasive probes comprises inserting the probes into a heart of the patient.

## Claims

1. A medical device, comprising:
an insertion tube, having a proximal end and a distal end, which is configured for insertion into a body of a patient;
a handle, which is fixed to the proximal end of the insertion tube and is configured to be held by a user of the device; and
a sensor associated with the handle and configured to generate a signal to a control console indicating whether the user is holding the handle.

2. The device according to claim 1, wherein the sensor comprises a flexible strain sensor that is fitted over an outer surface of the handle.

3. The device according to claim 1, wherein the sensor is configured to detect that the user is holding the handle responsively to movement of the handle.

4. The device according to claim 3, wherein the insertion tube contains a first magnetic field sensor in the distal end, for use in detecting a position of the distal end within the body responsively to a magnetic field applied from outside the body, and wherein the sensor in the handle comprises a second magnetic field sensor, which generates the signal in response to the magnetic field.

5. The device according to claim 1, wherein the insertion tube is configured for insertion into a heart of the patient, and the handle comprises one or more controls for operation by the user in manipulating the distal end of the insertion tube within the heart.

6. Medical apparatus, comprising:
a plurality of invasive probes, each such probe comprising:
an insertion tube, having a proximal end and a distal end, which is configured for insertion into a body of a patient;
a handle, which is fixed to the proximal end of the insertion tube and is configured to be held by a user of the probe; and
a sensor, which is configured to generate a signal indicating whether the user is holding the handle;
a display; and
a console, which is configured to present information on the display with respect to operation of the invasive probes inside the body and to provide, responsively to the signal from each probe, an indication as to which of the probes is currently being held by the user.

7. The apparatus according to claim 6, wherein the console is configured to present on the display an image of an organ in the body including symbols indicating respective positions of the probes in a vicinity of the organ, and wherein the indication comprises a change with respect to the symbols on the display to designate at least one of the probes that is currently being held by the user.

8. The apparatus according to claim 7, wherein the image comprises a three-dimensional view of the organ, and wherein the indication comprises rotating the three-dimensional view so as to enhance a visibility of a symbol corresponding to the at least one of the probes on the display.

9. The apparatus according to claim 7, wherein the image comprises a three-dimensional map of the organ, wherein the console is configured to detect position coordinates of the probes in the body and to generate the map based on the detected position coordinates of the at least one of the probes that is currently being held by the user.

10. The apparatus according to claim 6, wherein the indication comprises an audible notification identifying at least one of the probes that is being held by the user.

11. The apparatus according to claim 6, wherein at least one of the probes comprises a functional element configured to apply a treatment to tissue in the body, and wherein the console is configured to interrupt the treatment upon detecting that the user is not current holding the at least one of the probes.

12. The apparatus according to claim 11, wherein two or more of the probes are configured to apply the treatment, and wherein the console is configured to select one of the probes for application of the treatment by detecting that the one of the probes is currently being held by the user.

13. The apparatus according to claim 6, wherein the probes comprise a functional element configured to receive physiological signals from tissue in the body, and wherein the console is configured to present the physiological signals on the display, and wherein the indication comprises highlighting on the display at least one of the physiological signals that originates from one of the probes that is currently being held by the user.

14. The apparatus according to claim 6, wherein the probes comprise catheters for insertion into a heart of the patient.
